# EUROPEAN PATENT SPECIFICATION

(11) **EP 4 157 167 B1**
(45) Date of publication and mention of the grant of the patent: **29.05.2024**
(21) Application number: 21730096.1
(22) Date of filing: 27.05.2021
(51) Int. Cl.: A61F 5/443, A61F 5/445

(54) **AN ADHESIVE LAMINATE, A PRESSURE SENSITIVE ADHESIVE WAFER, AN OSTOMY APPLIANCE AND AN UROSTOMY DEVICE**
EIN KLEBENDES LAMINAT, EINE DRUCKEMPFINDLICHE KLEBEFOLIE, EINE STOMAVORRICHTUNG UND EINE UROSTOMIEVORRICHTUNG
UN LAMINÉ ADHÉSIF, UNE PLAQUETTE ADHÉSIVE SENSIBLE À LA PRESSION, UN APPAREIL POUR STOMIE ET UN DISPOSITIF POUR UROSTOMIE

(30) Priority: 29.05.2020 DK PA202070349
(43) Date of publication of application: 05.04.2023
(73) Proprietor: Coloplast A/S, 3050 Humlebaek (DK)
(72) Inventor: HANSEN, Kristoffer, 2850 Naerum (DK); HANSEN, Daniel, 2100 Copenhagen (DK); EILER, Johannes, 2860 Soeborg (DK)
(86) International application number: PCT/DK2021/050165
(87) International publication number: WO 2021/239198

(56) References cited:
- WO-A2-2008/154930
- WO-A2-2010/006600
- US-A1- 2016 143 768

## Description

An adhesive laminate, a pressure sensitive adhesive wafer, an ostomy appliance and an urostomy device

### Summary of the invention

In aspects, the invention relates to an adhesive laminate for application to skin, the laminate comprising a first, skin-facing adhesive layer and a second absorbent bulk layer. The skin-facing adhesive layer has a first proximal/skin-facing surface and a second distal surface and the bulk layer is provided on the second distal surface of the skin-facing adhesive layer.

### Brief Description of the Drawing

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Figure 1 shows a chart of peel force of four samples,
Figure 2 shows a chart of peel force of another three samples and
Figure 3 and Figure 4 show L/D90 versus water absorption.

### Detailed Description of the invention

In a first aspect, the present invention relates to an adhesive laminate as set out in claims 1-11.

In a second aspect, the present invention relates to a pressure sensitive adhesive wafer as set out in claim 12.

In a third aspect, the present invention relates to an ostomy appliance as set out in claim 13.

In a fourth aspect, the present invention relates to an urostomy device as set out in claim 14.

In the following Detailed Description, reference is made to the accompanying drawings which form a part hereof, and in which is shown by way of illustration specific embodiments in which the invention may be practiced. In this regard, directional terminology, such as "top," "bottom," "front," "back," "leading," "trailing," etc., is used with respect to the orientation of the Figure(s) being described. Because components of embodiments can be positioned in a number of different orientations, the directional terminology is used for purposes of illustration and is in no way limiting. It is to be understood that other embodiments may be utilized, and structural or logical changes may be made without departing from the scope of the present invention. The following detailed description, therefore, is not to be taken in a limiting sense, and the scope of the present invention is defined by the appended claims.

It is to be understood that the features of the various exemplary embodiments described herein may be combined with each other, unless specifically noted otherwise.

Throughout this disclosure, the words "stoma" and "ostomy" are used to denote a surgically created opening bypassing the intestines or urinary tract system of a person. The words are used interchangeably, and no differentiated meaning is intended. The same applies for any words or phrases derived from these, e.g. "stomal", "ostomies" etc. Also, the solid and liquid wastes emanating from the stoma may be referred to as both stomal "output," "waste(s)," and "fluids" interchangeably. A subject having undergone ostomy surgery may be referred to as "ostomist" or "ostomate" - moreover, also as "patient" or "user". However, in some cases "user" may also relate or refer to a health care professional (HCP), such as a surgeon or an ostomy care nurse or others. In those cases, it will either be explicitly stated, or be implicit from the context that the "user" is not the "patient" him- or herself.

In the following, whenever referring to proximal side of a device or part of a device, the referral is to the skin-facing side, when the ostomy appliance is worn by a user. Likewise, whenever referring to the distal side of a device or part of a device, the referral is to the side facing away from the skin, when the ostomy appliance is worn by a user. In other words, the proximal side is the side closest to the user, when the appliance is fitted on a user and the distal side is the opposite side - the side furthest away from the user in use.

The use of the word "substantially" as a qualifier to certain features or effects in this disclosure is intended to simply mean that any deviations are within tolerances that would normally be expected by the skilled person in the relevant field.

The use of the word "generally" as a qualifier to certain features or effects in this disclosure is intended to simply mean - for a structural feature: that a majority or major portion of such feature exhibits the characteristic in question, and - for a functional feature or an effect: that a majority of outcomes involving the characteristic provide the effect, but that exceptionally outcomes do not provide the effect.

The use of the word "essentially" as a qualifier to certain structural and functional features or effects in this disclosure is used for emphasizing what is the most important focus of something or fact about something (i.e. a feature may have or fulfil a variety of effects, but when the disclosure discusses one effect as being "essentially" provided, this is the focus and the most important effect in relation to the disclosure).

Throughout the disclosure, the use of the terms "first", "second", "third", "fourth", "primary", "secondary", "tertiary" etc. does not imply any particular order or importance but are included merely to identify individual elements. Furthermore, the labelling of a first element does not imply the presence of a second element and vice versa.

When addressing the thickness of the adhesive, the thickness is measured perpendicular to the skin-facing proximal surface, from this surface to the second distal surface.

When designing a skin adhesive, one of the major issues is to keep the skin relatively dry underneath the adhesive so as to prevent maceration. Maceration occurs when the skin is unable to get rid of moisture from for example transpiration and results in degradation of the skin's barrier function as well as bad adhesion of the device to the skin. Usually, skin adhesive keeps the skin dry by being moisture permeable. This allows moisture to transport through the adhesive from the skin side to the opposite side, where it is allowed to evaporate. Adhesives for fastening a bodily waste collecting pouch or tube may be covered by a moisture impermeable layer that does not allow moisture from the skin to permeate through the adhesive and out into the surroundings. Therefore, adhesives for fastening a collecting pouch or tube are made moisture absorbent. Absorbing particles or hydrocolloids (HC) are mixed into an adhesive sticky polymer matrix in order to absorb moisture from the skin and thereby keeping the skin relatively dry. This technique is well known in the art and forms the basis for most commercially available ostomy adhesives.

Conventional skin adhesives have a hydrocolloid content high enough to create a three-dimensional network of hydrocolloids where bodily fluids can be absorbed and stored. However, when the hydrocolloids particles at the skin-adhesive surface swell, they may displace and even deplete the sticky polymer matrix when absorbing water. Consequently, adhesion may be reduced when the adhesive is exposed to large volumes of bodily fluids for a prolonged time. If the hydrocolloid particle concentration in the adhesive is too low, the hydrocolloid particles are spaced too far away from each other's to be able to contact neighbouring particles and the adhesive will only absorb at the skin-facing surface resulting in a low absorbency and permeability of the adhesive as the hydrocolloid particles buried deeper in the adhesive sticky polymer matrix will not be able to get into contact with the moisture.

WO 2008/154930 discloses an adhesive laminate for adhesion to the skin.

So, there is still a need for an adhesive construction having high absorbency and an adhesive tack that is not compromised when the adhesive is exposed to moisture.

By isolating the highly absorbing part of the adhesive from the skin and introducing a layered construction with an absorbent bulk layer separated from the skin by a first skin-contacting adhesive layer capable of maintaining a proper adhesive tack when wetted, an adhesive laminate capable of absorbing considerable amounts of moisture and yet still maintain its adhesive properties even after moisture absorption is achieved.

The skin-facing adhesive layer may have a low hydrocolloid content so the concentration of hydrocolloid particles at the adhesive surface is low which allows more of the sticky polymer matrix to be present at the surface, thereby securing the adhesive properties and reduce hydrocolloid swelling near the skin-adhesive interface. However, effective water transport and a high water uptake capacity are still needed properties, which are typically achieved through a high hydrocolloid content. To achieve both these properties, the surface adhesive and the bulk adhesive compositions must be decoupled. This can be done using layered adhesive compositions.

The layer facing the skin will be referred to as the skin-facing adhesive layer, while the other layer will be referred to as the bulk layer. The bulk layer is located on the non-skin-facing surface of the skin-facing adhesive layer.

Thus, moisture permeation of the skin-facing adhesive layer can only be achieved through the particles as the sticky polymer matrix is impermeable to moisture. For moisture to permeate through the adhesive, particles need to touch each other. To achieve sufficient moisture permeability, lots of particles need to touch each other and this can only be achieved by mixing in large amounts of particles. These particles will absorb moisture and the entire adhesive will significantly change properties when exposed to water.

In a first aspect, the invention relates to adhesive laminate for application to skin, comprising a first, skin-facing adhesive layer and a second absorbent bulk layer, the skin-facing adhesive layer having a first proximal /skin-facing surface and a second distal surface, the bulk layer being provided on the second distal surface of the skin-facing adhesive layer, wherein the skin-facing adhesive layer has a first thickness, L, and comprises hydrocolloid particles having a particle size D90 being less than 290µm and wherein the ratio between the first thickness L and the particle size D90 is between 0.4-2.5.

In embodiments, the hydrocolloid content of the skin-facing adhesive layer is low in order to reduce the hydrocolloid swelling at the skin adhesive interface and allow more of the sticky polymer matrix to be present at the skin adhesive surface, which is expected to increase the adhesive tack.

The functionality of the skin facing adhesive layer is highly dependent on the first thickness/hydrocolloid particle size ratio. Here, we therefore define the parameter L/D90, where L is the first thickness and D90 is the particle size of the hydrocolloid particles.

In a cumulative particle size distribution, D90 describes the particle diameter where 90 % of all particles in the size distribution have diameters below D90. D90 is obtained through laser diffraction measurements. Method assumes that all particles are spherical. Method description: The particle size distributions were obtained using a Malvern Mastersizer 2000 with a dry dispersion unit (Scirocco 2000) (ISO13320 (2009)). Particles were dispersed at a pressure of 2.0 bar. D90: The portion of particles with diameters below this value is 90%. Hydrocolloid particles may be coarse particles having an irregular shape, but the above assumption still applies.

In embodiments, the ratio between the first thickness and the particle size D90 and is between 0.5 and 2.0, such as between 0.6 and 1.9, such as between 0.7 and 1.8, such as 0.8 and1.7. Having the first thickness close to the particle size of the hydrocolloid particles facilitates that the hydrocolloid particles may constitute a bridge over the first thickness of the skin-facing adhesive layer. In embodiments, the first thickness corresponds to the particle size D90 of the hydrocolloid particles. Larger particles can easier bridge through a thicker adhesive. In embodiments, the thickness/particle size ratio should be as close to 1 as possible, meaning that a single hydrocolloid particle will be able to bridge across the adhesive thickness and provide water transport through the skin-facing adhesive layer.

The major problem in using an absorbent adhesive is that the bonding properties change as the adhesive absorbs moisture. What started out as being a well-bonded adhesive, usually ends up being a weak-bonded adhesive after absorbing moisture. This effect is particularly a problem for hydrocolloid-based adhesives. These adhesives comprise two phases, a hydrophobic matrix (sticky polymer matrix) with hydrophilic hydrocolloids (HC) particles dispersed therein. As the adhesive absorbs moisture from the skin, the hydrocolloids swell and take up an increasing amount of space in the skin-bonding zone.

It is the hydrophobic part of the adhesive that is responsible for the bonding to the skin and as the hydrophobic part is being "squeezed" out by the expanding hydrophilic domains, the skin bonding is reduced. There is further a risk that, if small drops of moisture, e.g. due to active perspiration (sweating) are formed between the skin and the adhesive, small parts of hydrocolloid can be washed out into the droplet and thus reducing bonding between the skin and the adhesive. This creates an area in the skin-adhesive interface where no new adhesive bond can be formed as the HC itself has no cohesive and adhesive strength. If there had been no HC present, the adhesion would be better but then there would be no absorbing capacity of the adhesive.

To obtain effective water transport through the skin-facing adhesive layer both the hydrocolloid content and the first thickness to hydrocolloid size ratio is considered.

Effective liquid water/sweat transport through the skin-facing adhesive layer occurs:
In embodiments, the L/D90 is 0.4-1.8, such as 0.6-1.2. This may be advantageous when using low content of HC particles in the adhesive, where the distance between the particles may be high. Hence, in embodiments, the first thickness (L) is selected in such a way that the L/D90 ratio is between 0.6 and 1.2. Examples of low content of hydrocolloid may be 5-10 % w/w.

In embodiments, the L/D90 is 0.4-2.5, such as 0.8-2. This may be advantageous when using slightly higher content of HC particles in the adhesive, where the distance between the particles may be smaller. Examples of higher content of hydrocolloid may be 10-20 % w/w.

In embodiments, the hydrocolloid particles of the skin-facing adhesive layer have a D90 size below 290µm, such as below 270 µm, such as below 250 µm, such as below 230 µm, such as below 200 µm. In embodiments, the hydrocolloid particle size D90 is between 5-200 µm.

If the hydrocolloid particles of the skin-facing adhesive layer become too large, the adhesive may fail in handling moist conditions such as perspiration due to the large inter-particle distance (distance between the hydrocolloid particles). The interparticle distance is proportional to the particle diameter at fixed hydrocolloid weight fraction. - i.e. larger hydrocolloids = larger distance between them. The distance between hydrocolloids particles in the skin-facing adhesive layer should not be too high, since the chance of having a hydrocolloid near a sweat gland will be too low and adhesion may be compromised.

In embodiments, the thickness of the skin-facing adhesive layer is below 350 µm. in embodiments, the thickness is 30-150 µm.

The skin-facing adhesive layer comprises a sticky polymer matrix with hydrocolloid particles dispersed therein. In embodiments, the skin-facing adhesive layer comprises 5-20 % w/w hydrocolloid particles. The content of hydrocolloid particles secures a number of particles are present between the sweat glands at the skin surface ensuring that moisture from the pores is absorbed quickly.

Hydrocolloids which are useful in adhesives are well known in the art. Suitable water soluble and water swellable hydrocolloids may include carboxymethyl cellulose (CMC) (e.g. sodium carboxymethyl cellulose), pectin, guar gum, locust bean gum, gum karaya, Hydroxy ethyl cellulose etc. Polyacrylic acids known as superabsorbers can also be used. In embodiments, rheology modifiers such as potato starch or gelatin is used. They may help adjust the adhesion and flow without increasing water absorption significantly.

In embodiments, the sticky polymer matrix of the skin-facing adhesive layer is hydrophobic and has a low permeability to water vapor. In embodiments, the sticky polymer polymer matrix is vapor impermeable.

In embodiments, the sticky polymer matrix of the skin-facing adhesive layer is liquid permeable. In embodiments, the sticky polymer matrix is moisture impermeable.

In embodiments, the sticky polymer matrix is based on Polyisobutylene, Styrene-isoprene-styrene block copolymers, butylene rubber, styrene- isoprene deblock copolymer. In embodiments, plasticizing like DOA and softening oils like PI500 can be added. Tackifiers with different softening points may also be used. In embodiments, the sticky polymer matrix and the adhesive is thermoplastic to enable hotmelt processing.

In embodiments, rheology modifying particles such as for example potato starch or gelatine can be added to change mechanical properties of the adhesive without increasing absorption.

In embodiments, the skin-facing adhesive layer comprises 5-20 % w/w hydrocolloid, such as 5-15 % w/w hydrocolloid, such as 5-10% w/w hydrocolloid.

The low amount of hydrocolloid, compared to conventional hydrocolloid adhesives, facilitates less hydrocolloid elution/erosion, increased tack and thereby better adhesion during sweating or leakage.

The skin-facing adhesive layer is capable of maintaining adhesion during exposure to considerable amounts of liquid due to the low content of hydrocolloid. However, a certain amount of hydrocolloid content should be present, in order to secure effective liquid water transport through the skin-facing adhesive layer. Furthermore, to facilitate fast adhesion to wet skin, the adhesive should be able to absorb moisture.

In embodiments, the skin-facing adhesive layer has an absorption of at least 0.005 g/cm², such as at least 0.01 g/cm², such as at least 0.015 g/cm² or even at least 0.02 g/cm², measured as disclosed herein.

In embodiments, the skin-facing adhesive layer is vapor permeable. The presence of hydrocolloid particles facilitates the permeability.

In embodiments, the skin-facing adhesive layer is thinner than the bulk adhesive layer. In embodiments, the skin-facing adhesive layer is at least 10%, such as at least 20%, such as at least 30%, such as at least 40% or even at least 50% thinner than the bulk adhesive layer. The skin-facing adhesive layer may serve as a moisture transporting layer that moves the moisture into the absorbent bulk layer that is able to store considerable amounts of moisture.

In embodiments, the bulk layer has a thickness of 300-2000 µm, such as 400-1500 µm, such as 500-1200 µm.

In embodiments, the bulk layer comprises a hydrocolloid adhesive. In embodiments, the bulk layer comprises a 30-50 w/w% hydrocolloid. The bulk adhesive layer is provided on at least a part of the non-skin-facing surface of the skin-facing adhesive layer. In embodiments, the bulk layer may be similar in composition to conventional skin adhesives comprising a high hydrocolloid content to ensure three-dimensional hydrocolloid connectivity resulting in high fluid uptake capacity and effective fluid transport.

The bulk adhesive may comprise polyisobutylene, styrene-isoprene- styrene block copolymers, butylene rubber, styrene- isoprene deblock copolymer. In embodiments, plasticizing like DOA and softening oils like PI500 can be added. Tackifiers with different softening points may also be used. In embodiments, the bulk adhesive is substantially free from oil, such as paraffin oil.

In a second aspect, the invention relates to a pressure sensitive adhesive wafer for fastening a body waste collecting device to the skin. The body waste collecting device may be an ostomy pouch. In embodiments, the pressure sensitive adhesive wafer comprises a composite containing at least three layers: a adhesive laminate comprising a first, skin-facing adhesive layer and a second absorbent bulk layer, the skin-facing adhesive layer having a first proximal /skin-facing surface and a second distal surface, the bulk layer being provided on the second distal surface of the skin-facing adhesive layer, wherein the skin-facing adhesive layer has a first thickness, L, and comprises hydrocolloid particles having a particle size D90 being below 290 µm, and wherein the ratio between the first thickness L and the particle size D90 is between 0.4-2.5, and a backing layer. The backing layer may be provided on the distal surface of the laminate.

In embodiments, the invention relates to a pressure sensitive adhesive wafer for fastening a body waste collecting device to the skin. In embodiments, the body waste collecting device is an urostomy device. The adhesive laminate may facilitate of handling output/moisture of a wide range of different textures and viscosities.

### Methods

### Determination of water absorption:

In order to get better correlation between measured water absorption and actual performance in a humanlike environment, a modified version of the ISO 62 standard was used: Pieces of adhesive of 1x25x25 mm³ were fastened on a piece of glass using double sided adhesive and the constructs were immersed in 20 saline water (0.9% NaCl in demineralized water) at 32°C. After 2 hours, the samples were removed and carefully dripped dry and weighed. The change in weight was recorded and reported as weight gain in g/cm² of the adhesive.

### The perspiration peel test:

The adhesive performance under intense sweating conditions was evaluated by 90°-peel experiments from the perspiration simulator as previously published, Hansen, Daniel, et al. "Performance of Polymeric Skin Adhesives during Perspiration." ACS Applied Polymer Materials 2.4 (2020): 1535-1542.

Here, adhesives with dimensions of 100 × 25 × 1 mm³ (length × width × thickness) supplied with an acrylic backing tape were applied to the perspiration simulator with a pressure of 1680 Pa for 60 s. After application the adhesives were either peeled from the perspiration simulator immediately (0 min) or after 10 min of sweating + 10 minutes without sweating. The peel tests were done with constant displacement of 304 mm/min using an Instron 5943 Universal testing system with a 50 N load cell. The perspiration conditions were: 4.5 kPa, which yielded a perspiration rate of 1.6 ± 0.2 µL/min/cm² for uncovered artificial skin.

### The 'long-wear test':

The adhesive performance was tested under moderate sweating conditions for prolonged times by applying adhesives, with dimensions of 40 × 25 × 1 mm³ (length × width × thickness) and supplied with an acrylic backing tape, to the perspiration simulator. The adhesives were applied to the perspiration simulator with a pressure of 1680 Pa for 60 s. The perspiration simulator was subsequently hung upside-down, while a weight of 12 g was attached to one end of the adhesive. The perspiration simulator was sweating with a pressure of 3.5 kPa. The setup was filmed with a camera, which would detect the time at which the adhesive would fall off the sweating model. This time is referred to as the time of failure and is measured in minutes.

### Experimental

### TABLE 1

Bulk 1 (B-1): 40 % (w/w) CMC hydrocolloids dispersed in a 4/1 PIB/SIS (w/w) matrix.

Bulk 2 (B-2): 35% w/w mixed hydrocolloid (5 % pectin, 10 % CMC, 20 % Guar gum (w/w)) matrix.

S-1: 20% (w/w) CMC hydrocolloid dispersed in a 1/1 SIS/SI (w/w) matrix.

S-2: 20% (w/w) CMC hydrocolloid dispersed in a 4/1 PIB/SIS (w/w) matrix.

The skin-facing adhesive layer of sample A, B and C in Table 1 were laminated to a bulk adhesive layer to yield adhesive constructions with a total thickness of approximately 1000 µm. In Sample D, the entire sample is bulk layer with a thickness of 1000µm. The compositions of the layers are shown in Table 1.

**TABLE 1**

| **Sample** | **Bulk layer** | **Skin-facing adhesive layer** | **HC type** | **Content HC w/w %** | **L (µm)** | **D90 (µm)** | **L/D90** | **Dry peel (N)** | **Perspiration peel (N)** | **Time of failure (min)** |
|---|---|---|---|---|---|---|---|---|---|---|
| A | B-1 | S-1 | CMC | 20 | 50 | 97 | 0.52 | 19.3 | 25.0 | 1453 |
| B | B-1 | S-2 | CMC | 20 | 150 | 115 | 1.30 | 14.7 | 10.4 | 456 |
| C | B-2 | S-1 | CMC | 20 | 50 | 97 | 0.52 | 14.8 | 12.7 | 1008 |
| D | B-2 | | - | - | - | - | - | 0.7 | 1.3 | 273 |

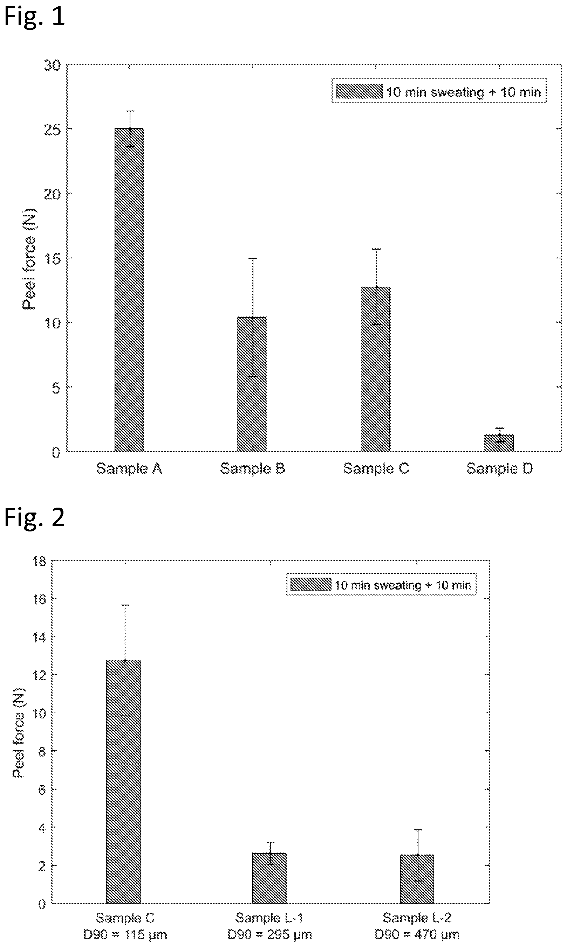

As can be seen from Table 1 and in Figure 1, the perspiration peels of the laminates (Sample A, B, C) are high. The perspiration peel of the reference (Sample D) is considerably lower than Sample A, B and C.

The time of failure is also considerably higher for the laminate of Sample A, B and C than for the reference in Sample D, showing that the laminate is also superior with regard to staying on the skin under moist conditions.

### Particle size

**TABLE 2**

| Sample | Bulk layer | First thickness (µm) (L) | Matrix chemistry | HC chemistry | HC content w/w (%) | D90 (µm) | Time of failure w. skin-facing adhesive layer (min) |
|---|---|---|---|---|---|---|---|
| L-1 | B-1 | 250 | PIB/SIS | PAA | 20 | 295 | 98 |
| L-2 | B-1 | 250 | PIB/SIS | CMC | 20 | 470 | 342 |
| L-3 | B-1 | 150, 250, 330, 450 | PIB/SIS | CMC (+Starch) | 20 (+19) | 115 | 602 |
| L-4 | B-1 | 125, 250, 400 | PIB/SIS | CMC | 10 | 115 | 456 |
| L-5 | B-1 | 50, 80, 150, 250 | SIS/SI | CMC | 20 | 97 | 1008 |

Figure 2 shows three adhesive samples tested under perspiration conditions. The D90 values represented the particle size in the skin facing layer. Sample L-1 held a skin-facing adhesive layer with first thickness, L = 250 µm, and PAA (poly acrylic acid) hydrocolloid content of 20 % (w/w) dispersed in a 4/1 PIB/SIS (w/w) matrix. Sample L-2 held a skin-facing adhesive layer with first thickness, L = 250 µm, and CMC content of 20 % (w/w) dispersed in a 4/1 PIB/SIS (w/w) matrix. Both Sample L-1 and Sample L-2 were laminated to a bulk layer of 800 µm thick B-1.

The laminates containing large hydrocolloids (Sample L1 and L2) failed the sweating tests due to the large inter-particle distance (distance between hydrocolloids). The interparticle distance is proportional to the particle diameter at fixed hydrocolloid weight fraction. - i.e., larger hydrocolloids = larger distance between them. It is important that the distance between hydrocolloids is not too long, since the chance of having a hydrocolloid near a sweat gland will be too low and adhesion will be compromised as seen in Figure 2.

### Upper limit to LID90

To investigate the overall requirement for water absorption of skin adhesives, to manage moderate perspiration conditions, adhesives were tested on the `long-wear test'. Here, an adhesive, Sample L-3, containing 20 % (w/w) CMC and 19 % starch (w/w) dispersed in a 4/1 PIB/SIS matrix, was investigated. The adhesive had a thickness of 1000 µm and proved to have a water absorption of 0.06 g/cm² after 2 hours of immersion. This adhesive had a *time of failure* of 86 min in the `long-wear test', which is unacceptable. A minimum requirement of water absorption of 0.07 g/cm² after 2 h is therefore set, showing the minimum value for effective moisture handling at the skin surface.

The 2h water absorption was measured for adhesives with different surface first thickness, L, and different hydrocolloid content to estimate the maximum ratio, L/D90, yielding a 2 h water absorption of at least 0.07 g/cm2.

### UD90

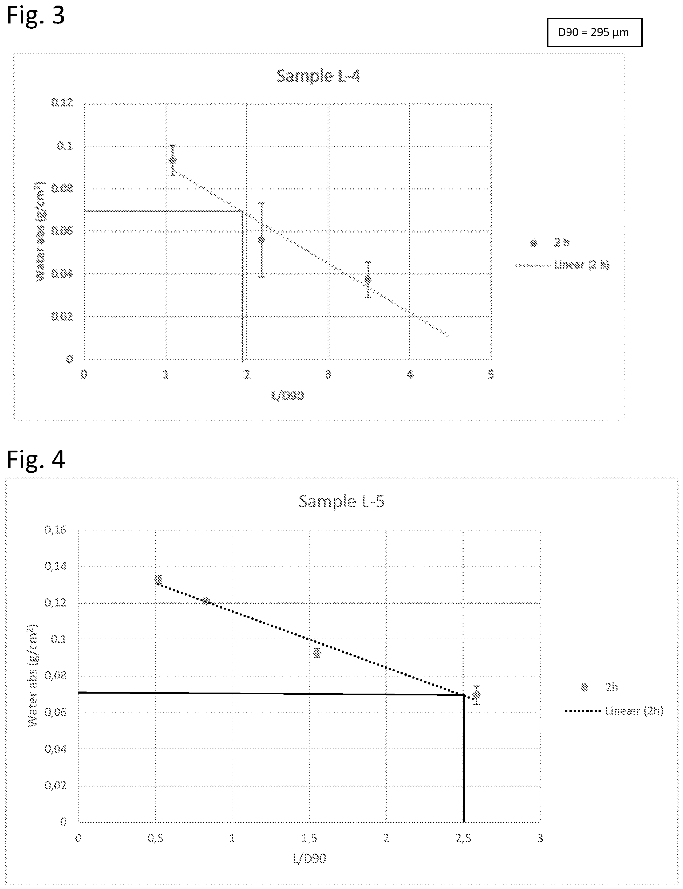

Sample L-4 consists of a skin-facing adhesive layer containing 10 % (w/w) CMC with D90 = 115 µm dispersed in a 4/1 PIB/SIS (w/w) matrix. The skin-facing adhesive layer was laminated with B-1 to yield adhesives with a total thickness of 1000 µm. The lines in Figure 3 indicates the minimum water absorption requirement and the maximum L/D90.

A similar approach was done for adhesive compositions with a skin-facing adhesive layer containing 20 % (w/w) CMC with D90 = 97 µm dispersed in a SIS/SI matrix. The skin-facing adhesive layer was laminated with B-1 to yield adhesives with a total thickness of 1000 µm. This laminate is referred to as Sample L-5 and the water absorption data of this composition is shown in the figure below where the lines indicate the minimum 2h water absorption and maximum L/D90 requirements.

Based on the 2h water absorption results of Sample L-4 and Sample L-5 and considering a margin of error, the requirements for the L/D90 are set to 0.4 < L/D90 < 2.5 for skin facing layers containing 10-20 % (w/w) hydrocolloids. In embodiments, the L/D90 is set to 0.4 < L/D90 < 1.8 for skin facing layers containing 5-10 % (w/w).

## Claims

1. An adhesive laminate for application to skin, comprising a first, skin-facing adhesive layer and a second absorbent bulk layer, the skin-facing adhesive layer having a first proximal, skin-facing surface and a second distal surface, the bulk layer being provided on the second distal surface of the skin-facing adhesive layer, wherein the skin-facing adhesive layer has a first thickness, L, and comprises hydrocolloid particles having a particle size D90 being less than 290µm and **characterized in that** the ratio between the first thickness L and the particle size D90 is between 0.6 and 1.2.

2. The adhesive laminate according to the preceding claims, wherein the hydrocolloid particles comprise carboxy methyl cellulose (CMC), pectin, guar gum, locust bean gum, gum karaya, hydroxy ethyl cellulose or polyacrylic acids.

3. The adhesive laminate according to the preceding claims, wherein the particle size D90 is between 5 and 200 µm.

4. The adhesive laminate according to the preceding claims, wherein the first thickness L is below 350 µm.

5. The adhesive laminate according to the preceding claims, wherein the skin-facing adhesive layer comprises 5-20 % w/w hydrocolloid.

6. The adhesive laminate according to the preceding claims, wherein the skin-facing adhesive layer has an absorption of at least 10 % w/w, measured as disclosed herein.

7. The adhesive laminate according to the preceding claims, wherein the skin-facing adhesive layer is thinner than the bulk layer.

8. The adhesive laminate according to the preceding claims, wherein the bulk layer comprises a hydrocolloid adhesive.

9. The adhesive laminate according to the preceding claims, wherein the bulk layer comprises a 20-45 w/w% hydrocolloid.

10. The adhesive laminate according to the preceding claims, wherein the bulk layer has a thickness of 300-2000 µm.

11. The adhesive laminate according to the preceding claims, wherein the adhesive laminate comprises a backing layer.

12. A pressure sensitive adhesive wafer for fastening a body waste collecting device to the skin comprising a composite containing at least three layers: an adhesive laminate comprising a first, skin-facing adhesive layer and a second absorbent bulk layer, the skin-facing adhesive layer having a first proximal /skin-facing surface and a second distal surface, the bulk layer being provided on the second distal surface of the skin-facing adhesive layer, wherein the skin-facing adhesive layer has a first thickness, L, and comprises hydrocolloid particles having a particle size D90 being less than 290µm and **characterised in that** the ratio between the first thickness L and the particle size D90 is between 0.6 and 1.2, and a backing layer.

13. An ostomy appliance comprising an adhesive laminate according to claims 1-11.

14. An urostomy device comprising an adhesive laminate according to claims 1-11.

## Patentansprüche

1. Haftfähiges Laminat zum Aufbringen auf Haut, umfassend eine erste hautzugewandte Haftschicht und eine zweite absorptionsfähige Masseschicht, wobei die hautzugewandte Haftschicht eine erste proximale hautzugewandte Oberfläche und eine zweite distale Oberfläche aufweist, wobei die Masseschicht an der zweiten distalen Oberfläche der hautzugewandten Haftschicht bereitgestellt ist, wobei die hautzugewandte Haftschicht eine erste Dicke L aufweist und Hydrokolloidpartikel mit einer Partikelgröße D90, die kleiner als 290 µm ist, umfasst und **dadurch gekennzeichnet ist, dass** das Verhältnis zwischen der ersten Dicke L und der Partikelgröße D90 zwischen 0,6 und 1,2 beträgt.

2. Haftfähiges Laminat gemäß den vorstehenden Ansprüchen, wobei die Hydrokolloidpartikel Carboxymethylcellulose (CMC), Pektin, Guargummi, Johannisbrotkernmehl, Karayagummi, Hydroxyethylcellulose oder Polyacrylsäuren umfassen.

3. Haftfähiges Laminat gemäß den vorstehenden Ansprüchen, wobei die Partikelgröße D90 zwischen 5 und 200 µm beträgt.

4. Haftfähiges Laminat gemäß den vorstehenden Ansprüchen, wobei die erste Dicke L unter 350 µm beträgt.

5. Haftfähiges Laminat gemäß den vorstehenden Ansprüchen, wobei die hautzugewandte Haftschicht 5-20 % Gew./Gew. Hydrokolloid umfasst.

6. Haftfähiges Laminat gemäß den vorstehenden Ansprüchen, wobei die hautzugewandte Haftschicht eine Absorption von wenigstens 10 % Gew./Gew., gemessen wie hierin offenbart, aufweist.

7. Haftfähiges Laminat gemäß den vorstehenden Ansprüchen, wobei die hautzugewandte Haftschicht dünner als die Masseschicht ist.

8. Haftfähiges Laminat gemäß den vorstehenden Ansprüchen, wobei die Masseschicht einen Hydrokolloidklebstoff umfasst.

9. Haftfähiges Laminat gemäß den vorstehenden Ansprüchen, wobei die Masseschicht 20-45 Gew./Gew.-% Hydrokolloid umfasst.

10. Haftfähiges Laminat gemäß den vorstehenden Ansprüchen, wobei die Masseschicht eine Dicke von 300-2000 µm aufweist.

11. Haftfähiges Laminat gemäß den vorstehenden Ansprüchen, wobei das haftfähige Laminat eine Trägerschicht umfasst.

12. Haftklebende Scheibe zum Befestigen einer Körperausscheidungssammelvorrichtung an die Haut, umfassend ein Verbundmaterial, das wenigstens drei Schichten enthält: ein haftfähiges Laminat, das eine erste hautzugewandte Haftschicht und eine zweite absorptionsfähige Masseschicht umfasst, wobei die hautzugewandte Haftschicht eine erste proximale/hautzugewandte Oberfläche und eine zweite distale Oberfläche aufweist, wobei die Masseschicht an der zweiten distalen Oberfläche der hautzugewandten Haftschicht bereitgestellt ist, wobei die hautzugewandte Haftschicht eine erste Dicke L aufweist und Hydrokolloidpartikel mit einer Partikelgröße D90, die kleiner als 290 µm ist, umfasst und **dadurch gekennzeichnet ist, dass** das Verhältnis zwischen der ersten Dicke L und der Partikelgröße D90 zwischen 0,6 und 1,2 beträgt, und eine Trägerschicht.

13. Stomavorrichtung, umfassend ein haftfähiges Laminat gemäß Ansprüchen 1-11.

14. Urostomavorrichtung, umfassend ein haftfähiges Laminat gemäß Ansprüchen 1-11.

## Revendications

1. Stratifié adhésif pour une application sur la peau, comprenant une première couche d'adhésif faisant face à la peau et une deuxième couche massive d'absorbant, la couche d'adhésif faisant face à la peau ayant une première surface proximale faisant face à la peau et une deuxième surface distale, la couche massive étant placée sur la deuxième surface distale de la couche d'adhésif faisant face à la peau, la couche d'adhésif faisant face à la peau ayant une première épaisseur, L, et comprenant des particules d'hydrocolloïde ayant une taille de particule D90 qui est inférieure à 290 µm et **caractérisé en ce que** le rapport entre la première épaisseur L et la taille de particule D90 est compris entre 0,6 et 1,2.

2. Stratifié adhésif selon les revendications précédentes, les particules d'hydrocolloïde comprenant une carboxyméthylcellulose (CMC), une pectine, une gomme de guar, une gomme de caroube, une gomme de karaya, une hydroxyéthylcellulose ou des poly(acide acrylique).

3. Stratifié adhésif selon les revendications précédentes, la taille de particule D90 étant comprise entre 5 et 200 µm.

4. Stratifié adhésif selon les revendications précédentes, la première épaisseur L étant inférieure à 350 µm.

5. Stratifié adhésif selon les revendications précédentes, la couche d'adhésif faisant face à la peau comprenant 5 à 20 % p/p d'hydrocolloïde.

6. Stratifié adhésif selon les revendications précédentes, la couche d'adhésif faisant face à la peau ayant une absorption d'au moins 10 % p/p, mesurée comme divulgué ici.

7. Stratifié adhésif selon les revendications précédentes, la couche d'adhésif faisant face à la peau étant plus fine que la couche massive.

8. Stratifié adhésif selon les revendications précédentes, la couche massive comprenant un adhésif d'hydrocolloïde.

9. Stratifié adhésif selon les revendications précédentes, la couche massive comprenant 20 à 45 % p/p d'hydrocolloïde.

10. Stratifié adhésif selon les revendications précédentes, la couche massive ayant une épaisseur de 300 à 2 000 µm.

11. Stratifié adhésif selon les revendications précédentes, le stratifié adhésif comprenant une couche de support.

12. Plaquette d'adhésif sensible à la pression pour la fixation d'un dispositif de collecte de déchets corporels sur la peau comprenant un composite contenant au moins trois couches : un stratifié adhésif comprenant une première couche d'adhésif faisant face à la peau et une deuxième couche massive d'absorbant, la couche d'adhésif faisant face à la peau ayant une première surface proximale/faisant face à la peau et une deuxième surface distale, la couche massive étant placée sur la deuxième surface distale de la couche d'adhésif faisant face à la peau, la couche d'adhésif faisant face à la peau ayant une première épaisseur, L, et comprenant des particules d'hydrocolloïde ayant une taille de particule D90 qui est inférieure à 290 µm et **caractérisée en ce que** le rapport entre la première épaisseur L et la taille de particule D90 est compris entre 0,6 et 1,2, et une couche de support.

13. Appareil de stomie comprenant un stratifié adhésif selon les revendications 1 à 11.

14. Appareil d'urostomie comprenant un stratifié adhésif selon les revendications 1 à 11.
